**Europäisches Patentamt**

**European Patent Office**

**Office européen des brevets**

(19)

(11) Veröffentlichungsnummer: **0 185 007**
**B1**

(12) # EUROPÄISCHE PATENTSCHRIFT

(45) Veröffentlichungstag der Patentschrift:
**18.01.89**

(51) Int. Cl.⁴: **C 11 D 3/12**, C 11 D 3/14,
C 11 D 7/20, A 61 L 2/00

(21) Anmeldenummer: **85890295.0**

(22) Anmeldetag: **28.11.85**

(54) **Mischung zur Pflege und Reinigung von Kontaktlinsen.**

(30) Priorität: **10.12.84 AT 3910/84**

(43) Veröffentlichungstag der Anmeldung:
**18.06.86 Patentblatt 86/25**

(45) Bekanntmachung des Hinweises auf die Patenterteilung:
**18.01.89 Patentblatt 89/3**

(84) Benannte Vertragsstaaten:
**BE CH DE FR GB IT LI LU NL SE**

(56) Entgegenhaltungen:
**EP-A- 0 063 472**
**GB-A- 2 055 118**
**US-A- 4 222 747**
**US-A- 4 394 179**

(73) Patentinhaber: **Koller, Anton, Operngasse 23,
A-1040 Wien (AT)**

(72) Erfinder: **Koller, Anton, Operngasse 23, A-1040 Wien
(AT)**

(74) Vertreter: **Beer, Otto, Dipl.-Ing. et al, Lindengasse 8,
A-1071 Wien (AT)**

# Beschreibung

Die Erfindung betrifft eine Mischung zur Pflege und Reinigung von Kontaktlinsen, enthaltend Wasser, ein abrasives Mittel auf Basis eines oder mehrerer Metalloxide mit einer Korngrösse kleiner als 10 µm und eines Suspensionshilfsmittels auf Basis quellfähiger Substanzen, wie Polyvinylalkohol, Zellulosederivaten, Guar-Gum sowie gegebenenfalls Puffermittel zur Einstellung des pH-Wertes auf einen Wert zwischen 5 und 8 und/oder Kochsalz, wobei in der Mischung das Verhältnis abrasives Mittel zu Wasser zwischen 1:99 und 90:10 liegt.

Unter Kontaktlinsen versteht man Sehhilfen, die beispielsweise aus durch Äthylenglykolmethacrylat quervernetztem Poly(Hydroxyäthylmethacrylat) oder Siloxanverbindungen oder auch Proteinverbindungen bestehen.

Harte Kontaktlinsen absorbieren kaum Wasser, wie dies bei den hydrophilen, weichen Kontaktlinsen der Fall ist, so dass die Verwendung herkömmlicher, aggressiver Reinigungs- und Desinfektionsmittel nach dem Gebrauch bei harten Kontaktlinsen kaum Probleme bereitet. So wurde aber beobachtet, dass Benzalkoniumchlorid oder Chlorbutanol enthaltende Pflegemittel für harte Kontaktlinsen hydrophobe Linsenoberflächen verursachen und insbesondere mit einigen siloxanhaltigen Linsenwerkstoffen nicht kompatibel sind.

Es ist weiters bekannt, dass die für harte Kontaktlinsen anwendbaren Reinigungs- und Pflegemittel bei weichen Kontaktlinsen nicht verwendet werden dürfen, da sich verschiedene, im Reinigungs- bzw. Pflegemittel enthaltene Wirkstoffe im Linsenwerkstoff anreichern. Die Folge ist eine irreversible Beschädigung des Linsenwerkstoffes sowie möglicherweise ernsthafte Schädigungen des Auges.

Dabei ist es aber unbestritten, dass es notwendig ist, Kontaktlinsen und somit auch weiche Kontaktlinsen zu reinigen. Die Kontaktlinsen schwimmen auf einem Tränenfilm, der aus einer die Hornhaut überdeckenden Schleimschicht, den eine wässerige Phase bildenden, eigentlichen Tränen und einem dünnen, öligem Film besteht. Die Schleimschicht hat die Aufgabe, die Hornhaut hydrophil und wasseranziehend zu machen, wogegen der ölige Film die Verdunstung des Tränenfilms verlangsamen soll. Die Schleimschicht besteht in erster Linie aus Proteinen, die von den verschiedenen Drüsen in den Augenliedern abgesondert werden. Wird nun eine Kontaktlinse aus dem Auge genommen, so bleiben auf ihr Eiweissreste haften, die in der Folge denaturieren und sich nur mehr schwer entfernen lassen. Diese Eiweissreste sind auch ein geeigneter Nährboden für Keime (Bakterien), so dass es notwendig ist, die Kontaktlinsen regelmässig von diesen Keimen und unerwünschten Rückständen zu befreien.

Die Reinigung von Kontaktlinsen auf rein chemischem Weg ist wegen der auf der Oberfläche derselben haftenden, gegebenenfalls mehr oder weniger denaturierten Eiweissrückstände praktisch nicht möglich.

Es sind daher die verschiedentlich vorgeschlagenen, rein chemischen Reinigungsmittel, die zum Teil auch mit unter der Wirkung eines Katalysators gespaltenem Wasserstoffperoxid arbeiten (vgl. EP-OS 0 082 798 oder US-PS 4 396 583), nicht hinreichend.

Es ist daher auch schon vorgeschlagen worden (vgl. US-PS 4 394 179 oder DE-OS 30 21 034), in Reinigungslösungen neben einem oberflächenaktiven Wirkstoff ein abrasives Mittel auf Basis von Metalloxiden zusammen mit einem Suspensionshilfsmittel in einer wässerigen Lösung oder einer organischen Flüssigkeit zu suspendieren. Die US-PS 4 394 179 schlägt dabei Teilchengrössen bis zu 10 µm vor.

Nachteilig bei dem aus der US-PS 4 394 179 bekannten Reinigungs- und Pflegemittel für Kontaktlinsen ist der Umstand, dass dieses jedenfalls einen oberflächenaktiven Wirkstoff enthalten muss und dass es in erster Linie nur für die Reinigung harter Kontaktlinsen geeignet ist.

Der Erfindung liegt die Aufgabe zugrunde, ausgehend von dem durch die US-PS 4 394 179 gegebenen Stand der Technik eine Mischung zur Reinigung und Pflege von Kontaktlinsen anzugeben, welche problemlos nicht nur für harte, sondern auch für weiche Kontaktlinsen verwendbar ist.

Erfindungsgemäss wird diese Aufgabe dadurch gelöst, dass das abrasive Mittel Zinkoxid und/oder Zinnoxid ist. Bevorzugt liegt die Korngrösse des abrasiven Mittels im Bereich von 0,5 µm.

Im Rahmen der Erfindung kann weiters vorgesehen sein, dass die Mischung Kochsalz in einer Menge enthält, bei welcher der osmotische Druck der Mischung dem osmotischen Druck von Tränenflüssigkeit im wesentlichen gleich ist.

Die erfindungsgemässe Mischung enthält keine Wirkstoffe, die in den Werkstoff weicher Kontaktlinsen eindringen und sich dort speichern und in der Folge das Auge schädigen könnten. Ein weiterer Vorteil der erfindungsgemässen Mischung liegt darin, dass bei Anwendung der erfindungsgemässen Mischung zur Reinigung (Reiben der Linsenoberfläche zwischen Fingerkuppen) im Werkstoff der Kontaktlinse keine tieferen Schleifspuren erzeugt werden, als sie herstellungsbedingt in den Oberflächen der Kontaktlinsen ohnehin vorhanden sind. Die erfindungsgemäss als abrasive Mittel vorgeschlagenen Metalloxide, nämlich Zinkoxid und/oder Zinnoxid sind Werkstoffe, die eine nur relativ geringe innere Härte aufweisen, so dass die Linsenoberfläche beim Reiben unter Anwendung der erfindungsgemässen Mischung weder beschädigt noch verändert wird. Ein wesentliches Merkmal der erfindungsgemässen Mischung liegt darin, dass das abrasive Mittel in einer einheitlichen Korngrösse vorliegt, die kleiner als 10 µm ist und bevorzugt in der Grössenordnung von 0,5 µm liegt.

Durch die Suspensionshilfsmittel, wie beispielsweise Hydroxyäthylzellulose, Xanthan-Gum, Guar-Gum, Polyvinylalkohol, Methylzellulose oder sonstige bekannte Extender wird die Suspension der abrasiven Teilchen im Wasser stabilisiert.

Durch Beigabe eines Pufferagens und/oder durch die Beigabe von Kochsalz in die Emulsion wird diese hinsichtlich des pH-Wertes und des osmotischen Druckes der Tränenflüssigkeit weitgehend angepasst.

Die erfindungsgemäss bevorzugte Verwendung von Zinkoxid als abrasives Mittel bringt zusätzlich den Vorteil mit sich, dass die Zinkionen durch die gering alkalische Wirkung eine teilweise Verseifung der Rückstände auf der Oberfläche der Kontaktlinse herbeiführen und sich so auch ohne Zusatz von oberflächenaktiven Stoffen eine schwache oberflächenaktive Wirkung ergibt.

Dadurch, dass beispielsweise durch die Zugabe von Natriumchlorid die erfindungsgemässe Mischung hinsichtlich ihres osmotischen Druckes nahe an die Eigenschaften einer physiologischen Kochsalzlösung herangebracht wird, ist gewährleistet, dass selbst bei unsachgemässer Anwendung, d.h. wenn die erfindungsgemässe Zinkemulsion nicht sorgfältig genug von der Kontaktlinse weggespült wurde, die Kontaktlinse sich am Auge nicht festsetzen kann.

Die in der erfindungsgemässen Mischung enthaltenen Metalloxide (Zinkoxid und/oder Zinnoxid), wobei ein Mischungsverhältnis von 1:99 (lösungsähnliches Mittel) bis 90:10 (pastöse Emulgat) verwendet wird, werden je nach der gewünschten Viskosität durch einen oder mehrere der oben erwähnten Extender in Suspension gehalten. Nach der Reinigung von harten und insbesondere weichen Kontaktlinsen durch Reiben derselben unter Anwendung der erfindungsgemässen Mischung können die Kontaktlinsen mit physiologischer Kochsalzlösung oder mit einem anderen Spülmittel von anhaftenden Resten der Mischung befreit werden. Anschliessend kann die Kontaktlinse, wie üblich, über Nacht gelagert werden.

Es ist bei Anwendung des erfindungsgemässen Mittels auch möglich, die Kontaktlinse nach dem Reiben und Anwenden der erfindungsgemässen Mischung nicht weiter abzuspülen, sondern unmittelbar über Nacht zu lagern. Diese Vorgangsweise hat den Vorteil, dass die oben geschilderte, verseifende Wirkung über Nacht und gegebenenfalls auch länger wirkt, wobei es dann genügt, kurz vor dem Gebrauch den Rückstand zusammen mit den Verunreinigungen unter Zuhilfenahme einer entsprechenden Spüllösung wegzuwaschen.

Die Anwendung der erfindungsgemässen Mischung zur Pflege und Reinigung von Kontaktlinsen kann selbstverständlich auch mit einem bekannten Reinigungsmittel, beispielsweise einem solchen auf Basis von Wasserstoffperoxid kombiniert werden. Hiezu kann man die mit der erfindungsgemässen Mischung abgeriebene Kontaktlinse einige Zeit, beispielsweise eine viertel Stunde bis zu 8 Stunden lang in eine Wasserstoffperoxidlösung mit einer Konzentration von 0,5% bis 30% einlegen. Durch die katalytische Wirkung des im erfindungsgemässen Mittel enthaltenen Zinkoxids oder Zinnoxids wird das Wasserstoffperoxid in Wasser und Sauerstoff gespalten, so dass man zusätzlich ein Sterilisieren der Kontaktlinse erreicht.

Es hat sich gezeigt, dass sich die erfindungsgemässe Mischung nicht nur zur Pflege weicher oder harter Kontaktlinsen eignet, sondern auch zur Reinigung hochgaslässiger harter Kontaktlinsen, wie z.B. solcher aus Materialien, welche zum Erzielen einer hohen Gasdurchlässigkeit einen hohen Siloxananteil haben. Dies sind beispielsweise die Materialien Boston II und Boston IV (Warenzeichen, Hersteller Polymer Technologies, Wilmington Mass, USA) oder Paraperm bzw. Paraperm EW (Warenzeichen, Hersteller Paragon, Mesa, Arizona, USA). Bei den vorerwähnten Kontaktlinsenwerkstoffen «Boston» und «Paraperm» handelt es sich um Fluor-Silikon-Methacrylate.

Um jedoch eine zusätzliche Reinigungswirkung zu erhalten, kann der erfindungsgemässen Mischung ein nichtionogenes Tensid, wie z.B. «Tego Betain HS» (ein Amphotensid auf Betain-Basis [Warenzeichen, Hersteller Th.Goldschmidt AG, Essen, BRD]) beigegeben werden. Dadurch wird auch eine bessere Benetzung der Kontaktlinsenoberfläche herbeigeführt.

Nachstehend wird ein Beispiel für die kombinierte Anwendung von Wasserstoffperoxid und der erfindungsgemässen Mischung angegeben:

Anwendungsbeispiel:

Die Kontaktlinse wird 10 Minuten bis 12 Stunden lang in eine 0,5 bis 30%-ige Wasserstoffperoxidlösung gelegt, danach mit 0,9%-iger isotonischer Kochsalzlösung abgespült und in ebenfalls 0,9%-iger isotonischer Kochsalzlösung gelagert. Hierauf wird 1 ml bis 10 ml der erfindungsgemässen Mischung (Emulsion) zugesetzt und die Kontaktlinse 1 Stunde bis 12 Stunden darin belassen. Zur Verbesserung der Reinigungswirkung empfiehlt es sich, die Kontaktlinse wenigestens einmal nach der Zugabe der erfindungsgemässen Mischung zwischen den Fingerkuppen leicht zu reiben, um die Reinigungswirkung zu erhöhen.

Nach beendeter Lagerung wird die gereinigte und sterilisierte Kontaktlinse mit 0,9%-iger Kochsalzlösung abgespült und kann unmittelbar verwendet werden.

Nachstehend werden einige Beispiele für erfindungsgemässe Mischungen, enthaltend Zinnoxid oder das bevorzugte Zinkoxid, angegeben.

Beispiel 1:
1 l destilliertes Wasser
9 g Kochsalz
73 g Zinkoxid
2,82 g Natrosol (nicht ionogene Hydroxyäthylzellulose Hersteller: Fa. Hercules in Rijswijk, NL)

Beispiel 2:
1 l destilliertes Wasser
1,5 g Xanthan-Gum (im Fermentationsverfahren hergestelltes Polysaccarid)
8 g Kochsalz
97 g Zinkoxid

Beispiel 3:
10 Gew.-% Zinkoxid
20 Gew.-% amphoteres Tensid (z.B. Tego Betain HS)
1,5 Gew.-% Guar-Gum
68,5 Gew.-% Aqua dest.

Beispiel 4:
15 Gew.-% Zinkoxid
15 Gew.-% amphoteres Tensid (z.B. Betain HS)
5 Gew.-% Natrosol
65 Gew.-% physiologische Kochsalzlösung

Da Verdickungsmittel, wie beispielsweise Xanthan-Gum in Wasser gelöst einen unangenehmen Geruch haben, ist es möglich, durch Beigabe von geringen Mengen Duftstoffen, wie beispielsweise Limettenöl, Pfefferminz- oder Rosmarinöl, eine Verbesserung des Geruches herbeizuführen.

## Patentansprüche

1. Mischung zur Pflege und Reinigung von Kontaktlinsen, enthaltend Wasser, ein abrasives Mittel auf Basis eines oder mehrerer Metalloxide mit einer Korngrösse kleiner als 10 μm, ein Suspensionshilfsmittel auf Basis quellfähiger Substanzen, sowie gegebenenfalls Puffermittel zur Einstellung des pH-Wertes auf einen Wert zwischen 5 und 8 und/oder Kochsalz, wobei in der Mischung das Verhältnis abrasives Mittel zu Wasser zwischen 1:99 und 90:10 liegt, dadurch gekennzeichnet, dass das abrasive Zinkoxid und/oder Zinnoxid ist.

2. Mischung nach Anspruch 1, dadurch gekennzeichnet, dass sie Kochsalz in einer Menge enthält, bei welcher der osmotische Druck der Mischung dem osmotischen Druck von Tränenflüssigkeit im wesentlichen gleich ist.

3. Mischung nach Anspruch 1 oder 2, dadurch gekennzeichnet, dass die Korngrösse des abrasiven Mittels im wesentlichen einheitlich ist und im Bereich von 0,5 μm liegt.

4. Mischung nach einem der Ansprüche 1 bis 3, dadurch gekennzeichnet, dass sie ein Tensid, vorzugsweise ein nichtionogenes oder amphoteres Tensid in einer Menge bis zu 25 Gew.-%, bezogen auf die Gesamtmischung, enthält.

## Claims

1. Mixture for the care and cleaning of contact lenses, containing water, an abrasive medium based on one or more metal oxides with a particle size less than 10 μm, a suspension promoting medium based on a swelling substance, and optionally buffer means for estabilishing the pH value at a value between 5 and 8 and/or common salt, wherein the ratio in the mixture of abrasive medium to water lies between 1:99 and 90:10, characterized in that the abrasive medium is zinc oxide and/or tin oxide.

2. Mixture according to claim 1, characterized in that the common salt is included in an amount at which the osmotic pressure of the mixture is substantially equal to the osmotic pressure of tears.

3. Mixture according to claim 1 or 2, characterized in that the particle size of the abrasive medium is substantially uniform and lies in the region of 0,5 μm.

4. Mixture according to one of claims 1 to 3, characterized in that it contains a surface active agent, preferably a non-ionic or amphoteric surface active agent in an amount up to 25% by weight, referred to the total mixture.

## Revendications

1. Mélange pour entretien et nettoyage de lentilles de contact, contenant de l'eau, un agent abrasif à base d'un ou plusieurs oxydes métalliques et ayant une dimension de particules inférieure à 10 μm, un adjuvant de suspension à base de substances susceptibles de gonfler, ainsi que, éventuellement, des agents tampon pour l'ajustement de la valeur du pH à une valeur comprise entre 5 et 8 et/ou du chlorure de sodium, le rapport du agent abrasif et de l'eau dans le mélange étant compris entre 1:99 et 90:10, caractérisé par le fait que l'agent abrasif est de l'oxyde de zinc et/ou de l'oxyde d'étain.

2. Mélange selon la revendication 1, caractérisé par le fait qu'il contient du chlorure de sodium en une quantité telle que la pression osmotique du mélange est pratiquement égale à la pression osmotique du liquide lacrymal.

3. Mélange selon les revendications 1 ou 2, caractérisé par le fait que la dimension des particules de l'agent abrasif est pratiquement homogène et qu'elle se situe aux alentours de 0,5 μm.

4. Mélange selon une des revendications 1 à 3, caractérisé par le fait qu'il contient un tensio-actif, de préférence un tensio-actif non ionogène ou amphotère, en une quantité allant jusqu'à 25% en poids par rapport à la quantité.